Europäisches Patentamt

European Patent Office

Office européen des brevets

(11) Publication number: **0 313 836**
**A2**

# EUROPEAN PATENT APPLICATION

(21) Application number: 88115832.3

(22) Date of filing: 26.09.88

(51) Int. Cl.⁴: **A61M 25/00** , **A61M 29/02** , **A61B 5/02**

(30) Priority: 30.09.87 US 103109

(43) Date of publication of application:
03.05.89 Bulletin 89/18

(84) Designated Contracting States:
CH DE FR GB IT LI NL

(71) Applicant: **ADVANCED CARDIOVASCULAR SYSTEMS, INC.**
**1395 Charleston Road P.O. Box 7101**
**Mountain View, CA 94039-7101(US)**

(72) Inventor: **Tremulis, William Stephen**
**97 Pelican Lane Redwood City**
**California 94065(US)**

(74) Representative: **Baillie, Iain Cameron et al**
**c/o Ladas & Parry Isartorplatz 5**
**D-8000 München 2(DE)**

(54) **Pressure monitoring guidewire.**

(57) Pressure monitoring guidewire and method of using the same for a coronary procedure such as angioplasty, angiography, or valvuloplasty. The guidewire has a hollow tubular member with a pressure monitoring port toward the distal end thereof. The distal end portion of the guidewire is advanced beyond the distal end of the dilatation catheter, with the pressure monitoring port in a portion of the coronary anatomy where pressure is to be monitored. With the guidewire so positioned, the pressure in the desired portion of the coronary anatomy is measured at the proximal end of the guidewire through the luminal opening in the hollow tubular member.

EP 0 313 836 A2

Xerox Copy Centre

# PRESSURE MONITORING GUIDEWIRE

## Background of the Invention

This invention pertains generally to coronary procedures such as angioplasty, angiography, and valvuloplasty, and more particularly to a pressure monitoring guidewire and a method of using the same.

Catheters have been employed in coronary procedures such as angioplasty and the like with means for taking pressure measurements. These catheters are usually advanced into the coronary anatomy along guidewires which pass through the lumen of the catheters through which the pressure measurements are made. The guidewires used with such catheters can interfere with the pressure measurements, and it is generally necessary to use a catheter of greater profile when pressure measurements are to be made.

The present invention, in one aspect thereof, provides a guidewire for use in a patient's blood vessel with a dilatation catheter having an inflatable balloon, comprising an elongated tubular member having proximal and distal portions and an internal lumen extending axially therethrough and at least one pressure monitoring port in the distal end portion thereof in fluid communication with the lumen to facilitate monitoring pressure in a portion of the anatomy beyond the distal end of the catheter.

In another aspect, the invention provides a dilatation catheter assembly comprising an outer tubular member having an inflatable balloon near the distal end thereof, an inner tubular member disposed within the outer tubular member, the distal ends of the inner and outer tubular members tightly secured together and a guidewire disposed within the inner tubular member having an elongated tubular member with proximal and distal portions and an internal lumen extending therethrough and at least one pressure monitoring port in the distal portion of the guidewire tubular member in fluid communication with the lumen therein to facilitate monitoring pressure beyond the distal end of the catheter.

In yet another aspect, the invention provides a method of monitoring pressure in a patient's blood vessel during a procedure such as angioplasty or valvuloplasty utilizing a dilatation catheter with an inflatable balloon on the distal portion thereof and a guidewire having an elongated hollow tubular member with at least one pressure monitoring port toward the distal end thereof, the method comprising the steps of: positioning the guidewire within the patient's blood vessels; advancing the catheter along the guidewire to position the balloon thereof adjacent to a lesion to be treated; positioning the guidewire relative to the catheter so that the pressure monitoring port is in a portion of the blood vessel beyond the distal end of the catheter, and monitoring the pressure at the proximal end of the hollow tubular member to determine the pressure in the portion of the blood vessel where the pressure monitoring port is located.

## Brief Description of the Drawings

FIGURE 1 is a fragmentary centerline sectional view of one embodiment of a pressure monitoring guidewire according to the invention;

FIGURES 2-6 are fragmentary centerline sectional views of additional embodiments of pressure monitoring guidewires according to the invention;

FIGURE 7 is a perspective view partially in section illustrating a low profile dilatation catheter with a guidewire embodying features of the invention; and

FIGURE 8 is a plan view of a dilatation guidewire and dilatation catheter system with a two-arm adapter.

## Detailed Description of the Invention

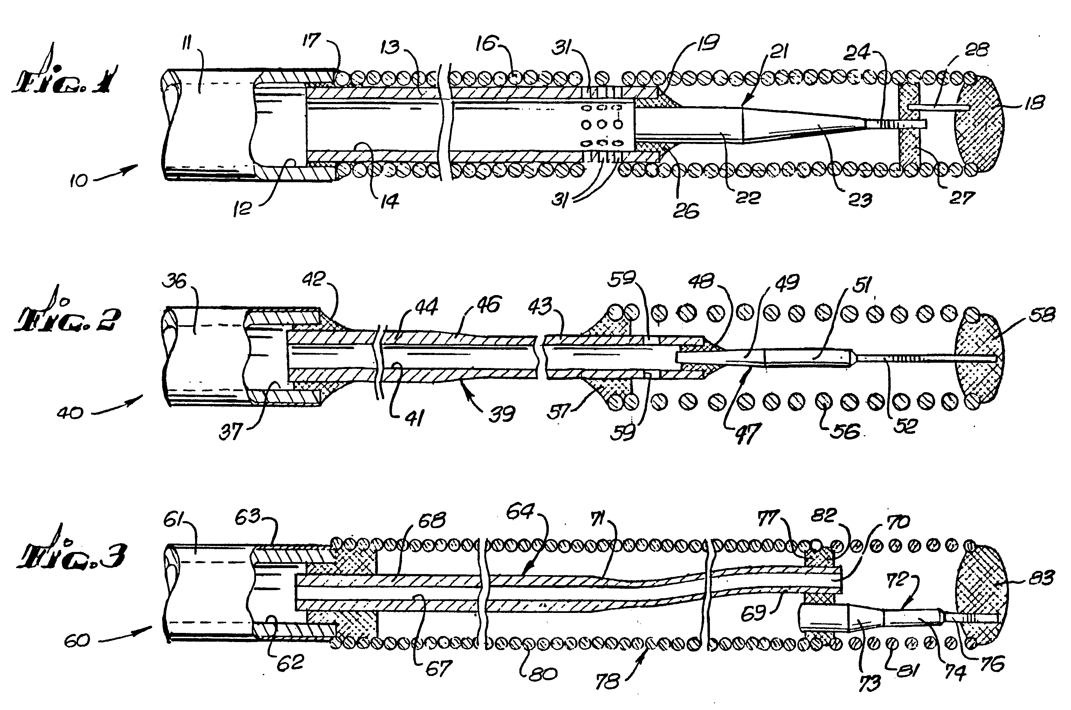

Figure 1 illustrates a guidewire 10 embodying features of the invention. As shown, the guidewire 10 has an elongated tubular shaft 11 which has an axially extending lumen 12. A tubular member 13 extends axially from the distal end of the tubular shaft 11, with a lumen 14 in axial alignment with the lumen 12 of the shaft 11. The tubular extension 13 has an outer diameter slightly smaller than the inner diameter of the shaft 11, and the proximal end portion of the tubular extension 13 is received within the distal end portion of the shaft 11.

A flexible spring coil 16 extends in an axial direction from the distal end of shaft 11 to form a flexible tip for the guidewire 10. Tubular extension 13 is positioned coaxially within the proximal end portion of the spring coil. The spring coil 16, the tubular shaft 11, and the extension 13 are bonded together by suitable means such as solder 17. A rounded tip of solder 18 is provided at the distal end of the coil.

A solid core 21 extends in an axial direction from the distal end of extension 13 within coil 16. The core 21 has a cylindrical proximal section 22, a conically tapered central section 23, and a flattened end section 24 of generally rectangular cross section. The proximal end portion of the core 21 is received within the distal end portion of the extension and affixed thereto by suitable means, such as solder 26. The flattened distal end portion 24 of the core 21 terminates prior to the distal end of coil 16, and the distal end of the core is affixed to the distal end portion of the coil by a solder disc 27. A safety wire 28 extends between the solder disc 27 and tip 18.

A plurality of pressure monitoring holes 31 are formed in the sidewall of tubular extension 13 near the distal end thereof. In the embodiment illustrated, the windings of coil 16 are spaced apart in the vicinity of the holes 31 to provide unobstructed access thereto. As shown, three axially spaced rows of holes 31 extend circumferentially around the tubular extension 13 and communicate with lumen 12 through lumen 14.

Holes 31 are intended primarily for use in monitoring pressure beyond the distal end of a dilatation catheter with which the guidewire is used.

Shaft 11 and extension 13 can be fabricated of any suitable material such as a hollow stainless steel tubing commonly known as hypodermic tubing or "hypo" tubing. Core 21 can likewise be fabricated of stainless steel or another suitable material, and coil 16 can be fabricated of stainless steel or, at least in part, a radiopaque material. Thus, in one presently preferred embodiment, coil 16 is fabricated of stainless steel on the proximal side of openings 31 and platinum on the distal side of the openings. The tapered portion 23 of core 21 with the safety wire 28 is only one possible configuration for the tip portion 19 of the guidewire 10. The distal portion of the guidewire 10 can have other suitable configurations.

In one presently preferred embodiment, the guidewire 10 has an overall length on the order of 175 cm, with shaft 11 being on the order of 145 cm long, holes 31 being spaced on the order of 25 cm from the distal end of the shaft 11, and coil 16 extending on the order of 5 cm beyond the holes 31. The spacing between the holes 31 and the distal tip 19 of the wire is preferably selected to position the holes distal to a lesion which is being dilated. For angioplasty use, the guidewire 10 has a diameter on the order of .25-.45 mm (.010-.018 inch), for angiography it can have a diameter on the order of .45-.965 mm (.018-.038 inch), and for valvuloplasty it can have a diameter on the order of .635-1.14mm (.025-.045 inch).

Operation and use of the guidewire and therein the method of the invention are as follows. A dilatation catheter having an inflatable balloon toward its distal end is inserted into the coronary anatomy through a guiding catheter and advanced into the desired artery along the guidewire. The guidewire is positioned so that holes 31 will be positioned beyond the distal end of the dilatation catheter when the balloon is in position to dilate a lesion. With the balloon inflated, the pressure on the distal side of the balloon can be monitored at the proximal end of the guidewire through holes 31 and luminal openings 12, 14.

A Tuohy-Bourst adapter may be attached to the proximal end of the guidewire shaft for attachment to a stopcock manifold. If an extension wire is needed, the Tuohy-Bourst adapter may be removed, and an extension shaft having a long tapered tip may be press-fit into the proximal end of lumen 12. The original catheter may then be removed and replaced with an alternate catheter. The extension wire is then removed, and the Tuohy-Bourst adapter is replaced on the proximal end of the wire for pressure monitoring.

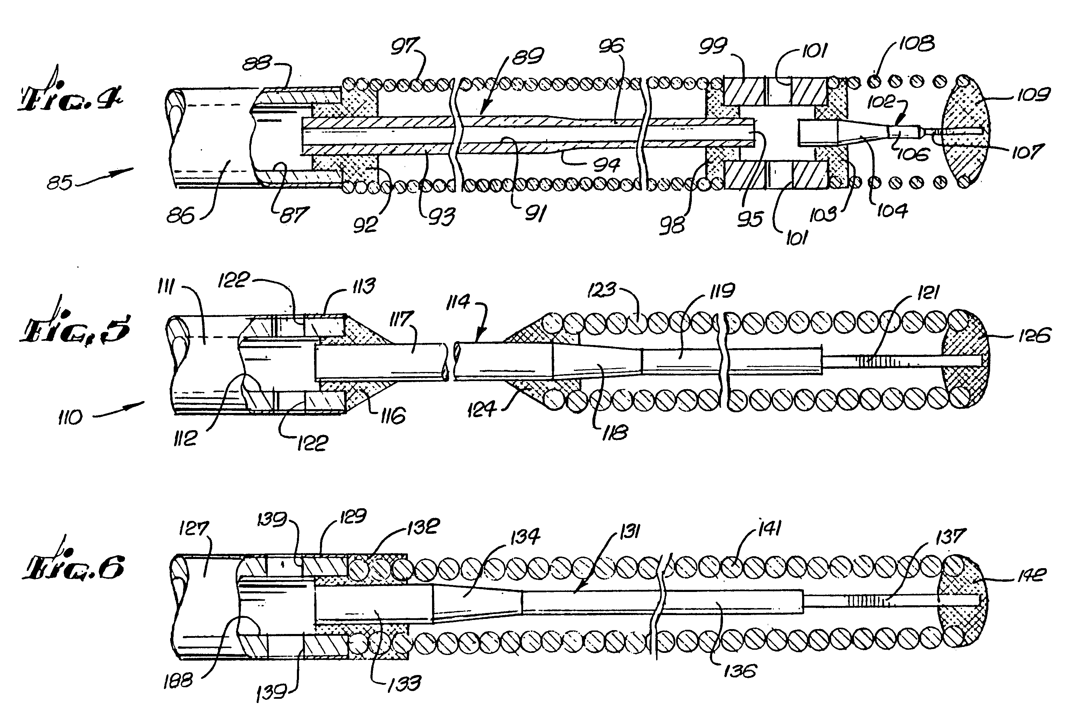

In the embodiment of Figure 2, guidewire 40 has an elongated tubular shaft 36 with an axially extending lumen 37 and an outer coating 38 of Teflon or other suitable lubricious material. A tubular member 39 extends axially from the distal end of the tubular shaft 36, with a lumen 41 in axial alignment with the lumen 37 of the shaft 36. The proximal end portion of the extension 39 is received within the distal end portion of the shaft 36 and is secured to the shaft 36 by solder 42. The distal section 43 of the extension 39 is of lesser diameter than the proximal section 44 and a tapered central section 46 is provided between the two sections.

A solid core 47 extends in an axial direction from the distal portion 43 of extension 39. The proximal end portion of the solid core 47 is received into lumen 41 in the distal section 43 of extension 39 and is secured therein by suitable means such as solder 48 which also closes the distal opening of lumen 41. The solid core 47 has a conically tapered proximal section 49, a cylindrical central section 51, and a flattened end section 52 of generally rectangular cross section.

A flexible spring coil 56 extends distally in an axial direction over part of extension 39 and core 47 and is preferably fabricated of a radiopaque material such as platinum. The proximal end of spring coil 46 is affixed to the extension 39 by solder 57, and the distal end of coil section 56 is secured to the distal end of the flattened tip section 52 of core 47 by solder 58, which forms a round tip for the guidewire 40.

A pressure monitoring hole 59 is formed in the sidewall of tubular extension 39 toward the distal end of the extension 39. This hole is located on the distal side of solder 57 and in an area in which the windings of spring coil 56 are spread apart.

In a guidewire intended for angioplasty use, for example, section 44 might have a diameter on the order of .20-.22 mm (.008-.0085 inch), section 43 might have a diameter on the order of .18-.19 mm (.0072-.0075 inch), and central section 46 might have a length on the order of 2.5-3.8 cm. Central lumen 41 has a uniform diameter on the order of .1 mm (.004 inch) throughout its length in this embodiment. Such a guidewire is particularly suitable for use with microdilation catheters such as the Hartzler® Micro™ manufactured and sold by the present applicant.

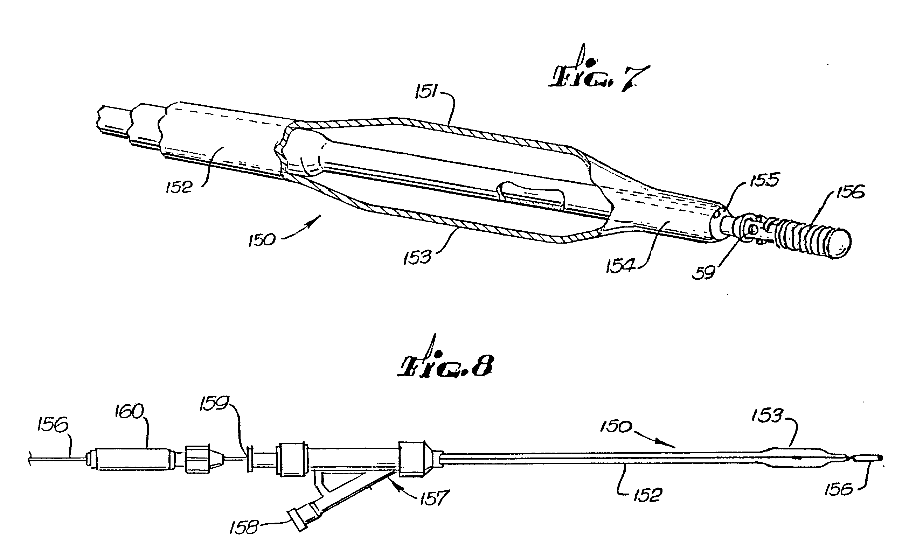

In the embodiment of Figure 3, the guidewire 60 has an elongated tubular shaft 61 with an axially extending lumen 62 and an outer coating 63 of Teflon or other lubricious material. A tubular member 64 extends axially from the distal end of the tubular shaft 61 and is affixed thereto by suitable means such as solder 66. The extension 64 has a lumen 67 which communicates with the lumen 62 in the shaft 61 and is open at its distal end to form a pressure monitoring hole 70. In the embodiment of Figure 3, extension 64 has a proximal section 68, a distal section 69 of lesser diameter than the proximal section, and a tapered central section 71 between the two sections. The distal portion of section 69 is offset laterally from the main axis of the guidewire 60.

A solid core 72 is positioned beside the distal end of extension 64 which extends to the distal end of the guidewire 60. The core 72 has a conically tapered proximal section 73, a cylindrical central section 74, and a flattened distal end section 76 of generally rectangular cross section. The proximal end portion of the solid core 72 is secured to the distal end portion of the extension by brazing 77. The core 72 is offset from the main axis of the guidewire 60, and it does not close the distal end of the lumen 62 in the extension 64 as in the previously described embodiments.

A spring coil 78 extends in an axial direction from the distal end of shaft 61 to the distal end of shaft 61 to the distal tip 83 of the guidewire 60. The coil has a proximal section 80 fabricated of stainless steel and a distal section 81 fabricated of a radiopaque material, such as platinum. The proximal end of coil section 80 is affixed to the shaft 61 and the extension 64 by solder 66, and the distal end of this coil section and the proximal end of coil section 81 are affixed to the extension and the core 72 by solder 82. The distal end of coil section 81 is affixed to the flattened distal tip section 76 of the solid core 72 by solder 83, which forms a rounded tip for the guidewire. The windings of coil section 81 are spaced apart to provide unobstructed access to the openings 70 at the distal end of extension 64.

In the embodiment shown in Figure 4, the guidewire 85 has an elongated tubular shaft 86 with an axially extending lumen 87 and an outer coating 88 of Teflon or other lubricious material. A tubular extension 89 extends in an axial direction from the distal end of the shaft 86, with a lumen 91 in communication with the lumen 87 opening in the shaft 86. As in the embodiment of Figure 3, the distal end of lumen 91 is provided with an opening 95. The extension 89 and the shaft 86 are affixed together by suitable means such as solder 92. The extension 89 has a cylindrical proximal section 93, a conically tapered central section 94, and a cylindrical distal section 96 of reduced diameter. A spring coil 97 which is fabricated of a material, such as stainless steel, extends coaxially of extension 89 from the distal end of shaft 86. The proximal end of the coil is affixed to the shaft 86 and to the extension 89 by solder 92, and the distal end of the coil 97 is affixed to the distal end portion of the extension 89 by solder 98.

A short tubular section 99 extends in an axial direction from the distal end of spring 97 to the tip 109. This tubular section is fabricated of a material, such as stainless steel, with a length on the order of 2.5 cm (one inch) and a diameter corresponding to the outer diameter of coil 97. Pressure monitoring holes 101 are formed in the sidewall of the short tubular section 99 on opposite sides thereof and in fluid communication with the luminal openings 95 in the tubular extension 89. The proximal end of tubular section 99 is affixed to coil 97 and extension 89 by solder 98.

A solid core 102 extends in an axial direction from the distal end of short tubular member 99 and is affixed thereto by solder 103. The core 102 has a conically tapered proximal section 104, a cylindrical central section 106, and a flattened distal section 107 of a generally rectangular cross section.

A spring coil 108 fabricated of a radiopaque material, such as platinum extends coaxially of core 102 from the distal end of tubular section 99. The proximal end of coil 108 is affixed to the tubular section and to the core by solder 103, and the distal end of this coil is affixed to the flattened distal end portion 107 of the core by solder 109 to form a round tip for the guidewire.

In the embodiment of Figure 5, the guidewire 110 has an elongated tubular shaft 111 with an axially extending lumen 112 and an outer coating 113 of Teflon or other lubricious material. The open distal end of the lumen 112 is closed by a solid core 114 which extends axially from the shaft 111 to the tip 126 and is affixed to the shaft 111 by solder 116. Solid core 114 has a cylindrical proximal section 117, a conically tapered section 118, a cylindrical section 119 of reduced diameter, and a flattened distal tip section 121 of generally rectangular cross section.

A pressure monitoring hole 122 is formed in the sidewall of tubular shaft 111 toward the distal end thereof and is in fluid communication with lumen 112.

A spring coil 123 extends coaxially of core 114. The proximal end of the coil is affixed to the core by solder 124, and the distal end of the coil is affixed to the flattened end section of the core by solder 126. The proximal end of coil 123 is spaced from the distal end of shaft 111 by a distance on the order of two inches.

In the embodiment of Figure 6, the guidewire has an elongated tubular shaft 127 with an axially extending lumen 128 and an outer coating 129 of Teflon or other lubricious material. As in the embodiment shown in Figure 5, a solid core 131 extends distally in an axial direction from the shaft 127 and closes the distal end of lumen 128. The core 131 is affixed to the shaft 127 by suitable means, such as solder 132. The core 131 has a proximal cylindrical section 133, an intermediate conically tapered section 134, a distal cylindrical section 136 of reduced diameter, and a flattened distal end section 137 of generally rectangular cross section.

A pressure monitoring hole 139 is formed in the sidewall of tubular shaft 127 toward the distal end thereof. This opening communicates with the lumen 128 in the shaft 127.

A coil spring 141 extends coaxially of core 131 from the distal end of the shaft 127 and is affixed to shaft 127 and the core 131 by solder 132. The distal end of the coil is affixed to the distal end portion of the core 131 by solder 142 to form a rounded tip for the guidewire.

Operation and use of the embodiments of Figures 2-6 are similar to that of Figure 1. A dilatation catheter having an inflatable balloon toward its distal end is inserted into the coronary anatomy through a guiding catheter over a guidewire of the invention and advanced into the desired artery along the guidewire. The guidewire is positioned so that the pressure monitoring holes are positioned beyond the distal end of the dilatation catheter when the balloon is in position to dilate a lesion. When the balloon is inflated for dilatation of the lesion, the pressure on the distal side of the balloon can be monitored at the proximal end of the guidewire through the pressure monitoring openings in the distal end of the guidewire. In addition, pressure measurements can be made proximal and distal to a stenosis prior to crossing of the stenosis with the balloon. The measurements made in this manner may indicate whether or not it is necessary to perform the angioplasty.

The guidewire of the invention has extensive application in procedures such as angioplasty, angiography, and valvuloplasty. A pressure gradient may be obtained between the guiding catheter at the coronary ostium and the tip of the guidewire on the distal side of the lesion before, during, and following dilatation. This wire eliminates the need for any pressure monitoring through the lumen of the dilatation catheter. This permits the catheter to be constructed with a lower profile, making it possible for the first time to monitor distal pressures through catheters of very low profile. Accurate pressure readings can be obtained regardless of whether the balloon is inflated or deflated. When the balloon is inflated, the guidewire will provide a "wedge pressure" reading for the coronary anatomy, possibly identifying the presence of collateral circulation distal to the lesion or identifying other circulatory anomalies. The guidewire is highly torquable and is readily steered to facilitate placement in the cardiovascular system.

Figure 7 illustrates a guidewire in accordance with the invention disposed within a low profile dilatation catheter such as the Hartzler® MicroTM dilatation catheter manufactured and sold by the present applicant, Advanced Cardiovascular Systems, Inc. The catheter 150 shown generally comprises an inner tubular member 151, and an outer tubular member 152 having an inflatable balloon element 153 adjacent the distal end thereof. The distal end 154 of the outer tubular member 152 is preferably shrunk-fit or otherwise tightly secured to the distal end 155 of the inner tubular member 151. Guidewire 156, such as that shown in Figure 2, is disposed within the lumen of the inner tubular member 151.

Figure 8 represents a dilatation catheter system including a catheter 150 and guidewire 156 shown in Figure 7. A two-arm adapter 157 is provided at the proximal end of the catheter 150, having an inflation port 158 and a guidewire port 159. A torque device 160 is provided on the proximal end of the guidewire 156.

The wire may also be used to deliver therapeutic agents to a specific area or organ, such as streptokinase or TPA to a lesion during acute myocardial infarction (heart attack), or chemotherapeutic drugs to a tumor. A heparin drip may be employed to eliminate any clotting of blood within the fluid passageways of the wire.

It is apparent from the foregoing that a new and improved pressure monitoring guidewire and method using the same has been provided. While only certain presently preferred embodiments have been described in detail, as will be apparent to those familiar with the art, certain changes and modifications can be made without departing from the scope of the invention as defined by the following claims.

## Claims

1. A guidewire for use in a patient's blood vessel with a dilatation catheter having an inflatable balloon, comprising an elongated tubular member having proximal and distal portions and an internal lumen extending axially therethrough and at least one pressure monitoring port in the distal end portion thereof in fluid communication with the lumen to facilitate monitoring pressure in a portion of the anatomy beyond the distal end of the catheter.

2. The guidewire of claim 1 wherein the pressure monitoring ports open through the sidewall of the tubular member.

3. The guidewire of any one of the preceding claims wherein the tubular member comprises a tubular shaft and a relatively short tubular extension at the distal end of the tubular shaft with at least one pressure monitoring port formed in the tubular extension.

4. The guidewire of claim 3 including a solid core extending axially from the distal end of the tubular extension to the distal end of the guidewire.

5. The guidewire of claim 4 wherein a flexible spring coil extends proximally from the distal end of the guidewire and is disposed about the tubular extension and the solid core, the windings of the coil in the region of at least one pressure monitoring port being spaced apart to provide unobstructed fluid access thereto.

6. The guidewire of claim 4 wherein the tubular shaft has a length on the order of 145 cm, the pressure monitoring ports are positioned about 25 cm beyond the distal end of the tubular shaft, and the spring coil extends a distance on the order of 5 cm beyond the pressure monitoring hole.

7. The guidewire of claim 3 wherein an intermediate tubular member having at least one port in the sidewall thereof is secured to the distal end of the tubular extension.

8. The guidewire of claim 1 wherein a solid core extends from the distal end of the tubular member to the distal end of the guidewire.

9. The guidewire of claim 8 wherein a flexible spring coil extends proximally from the distal end of the guidewire and is disposed about the solid core.

10. A dilatation catheter assembly comprising an outer tubular member having an inflatable balloon near the distal end thereof, an inner tubular member disposed within the outer tubular member, the distal ends of the inner and outer tubular members tightly secured together and a guidewire disposed within the inner tubular member having an elongated tubular member with proximal and distal portions and an internal lumen extending therethrough and at least one pressure monitoring port in the distal portion of the guidewire tubular member in fluid communication with the lumen therein to facilitate monitoring pressure beyond the distal end of the catheter.

11. A method of monitoring pressure in a patient's blood vessel during a procedure such as angioplasty or valvuloplasty utilizing a dilatation catheter with an inflatable balloon on the distal portion thereof and a guidewire having an elongated hollow tubular member with at least one pressure monitoring port toward the distal end thereof, the method comprising the steps of:
positioning the guidewire within the patient's blood vessels;
advancing the catheter along the guidewire to position the balloon thereof adjacent to a lesion to be treated;
positioning the guidewire relative to the catheter so that the pressure monitoring port is in a portion of the blood vessel beyond the distal end of the catheter, and monitoring the pressure at the proximal end of the hollow tubular member to determine the pressure in the portion of the blood vessel where the pressure monitoring port is located.

12. The method of claim 11 including the step of introducing a therapeutic agent into the blood vessel through the hollow tubular shaft and the pressure monitoring port of the guidewire.

FIG. 1

FIG. 2

FIG. 3

EP 0 313 836 A2

Fig.4

88
97
89
96
99
101
108
85
86
87
92
93
91
98
95
103
104
101
94
102
106
107
109

Fig.5

111
122
113
117
114
123
119
121
112
116
124
118
122
110
126

Fig.6

127
139
129
132
134
131
141
137
188
139
133
136
142

Not sufficiently fixed / Neuroscan Corpse

EP 0 313 836 A2

**Fig. 7**

151

152

155

156

150

153

59

154

**Fig. 8**

156

160

159

150

153

152

156

157

158